(19) Europäisches Patentamt
European Patent Office
Office européen des brevets

(11) **EP 4 096 861 B1**

(12) **EUROPEAN PATENT SPECIFICATION**

(45) Date of publication and mention
of the grant of the patent:
**20.03.2024 Bulletin 2024/12**

(21) Application number: **20712678.0**

(22) Date of filing: **27.01.2020**

(51) International Patent Classification (IPC):
**B23K 31/12** *(2006.01)*     **B23K 26/03** *(2006.01)*

(52) Cooperative Patent Classification (CPC):
**B23K 31/125; B23K 26/034;** Y02E 60/10

(86) International application number:
**PCT/IB2020/050602**

(87) International publication number:
**WO 2021/152344 (05.08.2021 Gazette 2021/31)**

(54) **METHOD OF MONITORING THE QUALITY OF A WELD BEAD, RELATED WELDING STATION AND COMPUTER-PROGRAM PRODUCT**

VERFAHREN ZUR ÜBERWACHUNG DER QUALITÄT EINER SCHWEISSRAUPE, ZUGEHÖRIGE SCHWEISSSTATION UND COMPUTERPROGRAMMPRODUKT

PROCÉDÉ DE SURVEILLANCE DE LA QUALITÉ D'UN CORDON DE SOUDURE, POSTE DE SOUDAGE ET PRODUIT DE PROGRAMME INFORMATIQUE ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.12.2022 Bulletin 2022/49**

(73) Proprietor: **Comau S.p.A.**
**10095 Grugliasco (Torino) (IT)**

(72) Inventors:
• **DI STEFANO, Giovanni**
**10095 Grugliasco Torino) (IT)**
• **LONGO, Nicola**
**10095 Grugliasco Torino) (IT)**

(74) Representative: **Meindl, Tassilo**
**Buzzi, Notaro & Antonielli d'Oulx S.p.A.**
**Corso Vittorio Emanuele II, 6**
**10123 Torino (IT)**

(56) References cited:
**DE-A1-102008 058 422     DE-A1-102013 112 244**
**US-A1- 2005 169 346**

**Description**

Technical field

The embodiments of the present description relate to techniques for monitoring the quality of a weld.

Background

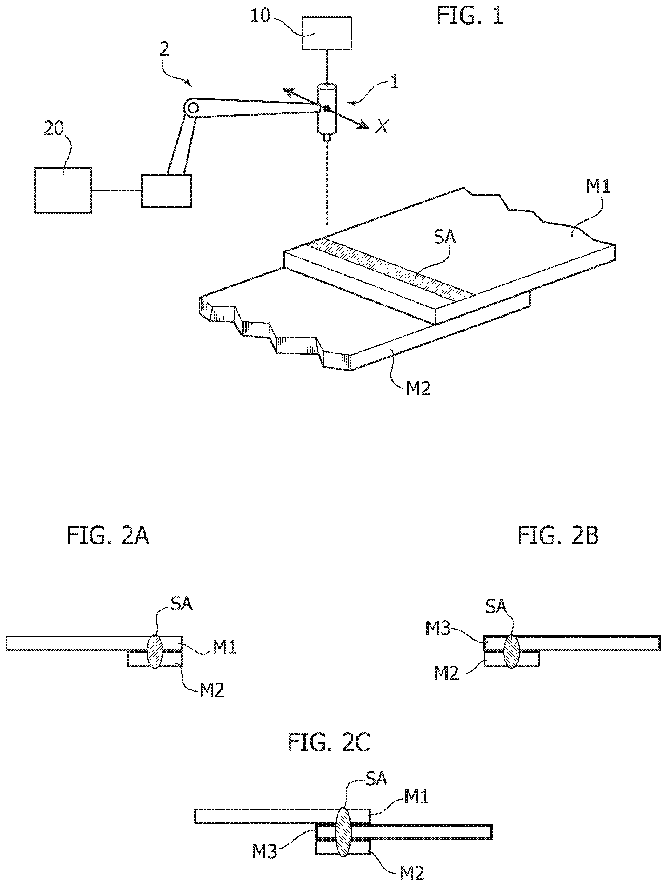

Figure 1 shows a typical welding station of an industrial plant.

In the example considered, welding is used to join two or more metal pieces M1 and M2 along a welding path. For instance, Figure 1 illustrates two plates set on top of one another, M1/M2, and the weld should be made along a known trajectory in a direction designated by $x$.

In particular, in the example considered, welding is carried out by means of an energy source that comprises a welding head 1, such as an electron source for electron-beam welding (EBW) or a photon source, typically a laser source. Typically, associated to the source and/or the welding head 1 is a control circuit configured for regulating one or more parameters of the source and/or of the welding head 1, such as the power emitted by the source or focusing of the beam emitted by the welding head, etc.

In addition, the welding station comprises at least one actuator 2 for moving the beam emitted by the welding head 1 along the welding path. For instance, this can be obtained by turning the welding head 1 and/or (as illustrated in Figure 1) by displacing the welding head 1 with respect to the pieces M1 and M2 and/or by moving at least one axis of the optical chain. For instance, Figure 1 shows, for this purpose, a robot arm 2. Hence, typically, also the actuator or actuators 2 has/have associated thereto a control circuit 20 configured for driving the actuator or actuators 2 in order to move the beam emitted by the welding head 1 along the welding path.

Consequently, the electron beam or photon beam generated by the source and emitted by the welding head 1 strikes the top piece M1 along the welding path and melts the materials of the pieces M1 and M2 in a welding zone SA, thus obtaining a weld bead. In general, the metal pieces M1 and M2 may have any shape, and it is sufficient for the pieces M1 and M2 to be in contact, i.e., to have complementary shapes, along the welding path, in the welding zone SA. For this purpose, blocking/gripping means are typically used, which are configured for blocking the pieces M1 and M2, in particular in the welding zone SA, in such a way as to guarantee an appropriate contact between the pieces M1 and M2.

Furthermore, the pieces M1 and M2 may also be made of different materials. For instance, this is typically the case when batteries, in particular for electric vehicles, are to be produced. For instance, for this purpose, reference may be made to the documents Nos. US 2015/0207127 A1 and US 2017/0341144 A1 or the paper by Das, A.; Li, D.; Williams, D.; Greenwood, D., "Joining Technologies for Automotive Battery Systems Manufacturing", World Electr. Veh. J. 2018, 9, 22.

For instance, in this case, welding can be used for connecting a first tab to a busbar and/or to a second tab. For instance, this is schematically illustrated in Figures 2A to 2C. In particular, Figure 2A is a cross-sectional view, where a first tab M1, for example made of aluminium (Al), is welded to a busbar M2, for example made of copper (Cu). Likewise, Figure 2B is a cross-sectional view, where a second tab M3, for example made of nickel (Ni) or copper (Cu), is welded to the busbar M2. Finally, Figure 2C is a cross-sectional view, where the first tab M1 is welded to the second tab M3, and possibly also to the busbar M2. For instance, the aforesaid tabs and busbar may have a thickness of between 0.3 and 0.8 mm.

Even though modern welding stations meet stringent criteria of quality and stability over time, a check on the quality of the weld may be required. For instance, this is particularly important in the context of batteries for the automotive sector since such batteries must have a uniform electrical resistance between the tabs and the busbars.

The person skilled in the art will appreciate that for continuous monitoring of the weld quality a non-destructive testing method is typically called for. In particular, such non-destructive tests are experimental investigations aimed at identifying and characterizing any discontinuities in the weld bead that might potentially jeopardize the performance thereof in the end product. The point in common with non-destructive testing techniques is hence their capacity not to affect in any way the chemical, physical, and functional characteristics of the object under analysis. For instance, in this context, reference may be made to the UNI EN 473 standard.

Accordingly, the present invention relates to a method of analysing the quality of a weld bead according to the preamble of Claim 1, which is known, e.g., from DE 10 2013 112244 A1.

Summary

The object of the invention is hence new solutions that enable monitoring of the quality of a weld bead.

According to the invention, this object is achieved via a method having the distinctive elements set forth specifically in the ensuing claims. The embodiments of the invention moreover regard a corresponding welding system according to claim 10, as well as a corresponding computer-program product according to claim 11. As used herein, reference to such a computer-program product is intended as being equivalent to reference to a computer-readable medium containing instructions for controlling a computing system in order to co-ordinate execution of the method. Reference to "at least one computer" is intended to highlight the possibility of the present invention being implemented in a distributed/modular way.

The claims form an integral part of the technical teaching of the disclosure provided herein.

As explained previously, the invention relates to a method for analyzing the quality of a weld bead in a welding zone. The weld bead is generated by means of a continuous welding operation, in which an energy beam emitted by a source with corresponding welding head follows a welding path, thereby melting the material of at least two metal pieces.

According to the invention, the welding zone is monitored via a thermal camera. In particular, the thermal camera supplies a sequence of thermal images/frames in which a given area corresponds to the welding zone. For instance, this area may be determined while performing a welding operation. For example, a rectangular or trapezoidal area may be defined in the thermal image as region of interest. On the hypothesis of an automatic search for the region of interest, starting from an observation window of fixed and preset dimensions, the processing circuit 30 can position the region of interest in the image by maximizing the functional represented by the sum of the temperatures of the pixels included therein for all the frames.

According to the invention, the above area is divided into a plurality of sub-areas, and for each sub-area a respective temperature is determined as a function of the values of the pixels within the respective sub-area. For instance, the temperature of a given sub-area may be determined via the mean or a weighted mean of the values of the pixels within the respective sub-area.

During a learning step, a plurality of welding operations are carried out, in particular at least for a plurality of examples in which the weld has a sufficient quality and a plurality of examples in which the process is not of good quality. In addition, the temperature evolution of each sub-area is monitored during each welding operation.

During a training step, the temperature evolutions monitored during the learning step are processed for training a classifier. For instance, for this purpose, an operator can classify the quality of the welds; i.e., the system can receive (from the operator), for each weld, data that identify the respective weld quality. Consequently, the classifier is configured for estimating a weld quality as a function of respective temperature evolutions.

In particular, a respective cooling curve is extracted from each temperature evolution, and, for each cooling curve, parameters are determined that identify the shape of the cooling curve. Consequently, these parameters are used as input features for the classifier.

For instance, in various embodiments, the shape of the cooling curve is, for this purpose, approximated via interpolation with a function made up of a plurality of base functions, thereby selecting, for each base function, a respective set of parameters. Consequently, in this case, the parameters of the interpolation may be used as input features for the classifier. For example, in various embodiments, an exponential interpolation is used.

Instead, during a normal welding operating step, the temperature evolution of each sub-area can be monitored again during execution of one or more welding operations, and the respective weld quality can be estimated by means of the classifier that has previously been trained. For instance, the classifier may be an artificial neural network. In general, the same classifier or a further classifier can also be used for estimating a defective-weld class.

In general, the classifier may also receive one or more further input features, such as the peak of each temperature evolution, the power emitted by the source, the speed of advance with which the energy beam follows the welding path, etc.

Brief description of the drawings

The embodiments of the present disclosure will now be described with reference to the annexed drawings, which are provided purely by way of non-limiting example and in which:

- Figure 1 shows an example of a welding station;
- Figures 2A to 2C show some examples of welding operations;
- Figure 3 shows an embodiment of a welding station that comprises a thermal camera;
- Figure 4 shows an embodiment, in which the welding station of Figure 3 melts two materials in a welding zone;
- Figure 5 shows an example of the image of the welding zone, as captured by the thermal camera of Figure 3;
- Figure 6 shows an embodiment of a segmentation of the welding zone into a number of sub-areas;
- Figure 7 shows an example of the temperature evolutions of the sub-area of Figure 6;
- Figure 8 shows an embodiment for extracting a cooling curve from a respective temperature evolution;
- Figure 9 shows an embodiment for determining a weld quality as a function of the cooling curves extracted;
- Figures 10 and 11 show embodiments of the classifier used in Figure 9; and

- Figure 12 shows an embodiment for training and use of the classifier of Figure 9.

Detailed description

In the ensuing description, numerous specific details are provided for enabling an in-depth understanding of the embodiments. The embodiments may be implemented without one or more specific details, or with other methods, components, materials, etc. In other cases, well-known operations, materials, or structures are not represented or described in detail so that aspects of the embodiments will not be obscured.

Throughout the present description, reference to "an embodiment" or "one embodiment" means that a particular characteristic, distinctive element, or structure described with reference to the embodiment is comprised in at least one embodiment. Thus, phrases such as "in an embodiment" or "in one embodiment" that may appear in various points of this description do not necessarily all refer to one and the same embodiment.

The references used herein are provided simply for convenience and consequently do not define the sphere of protection or the scope of the invention as defined by the claims.

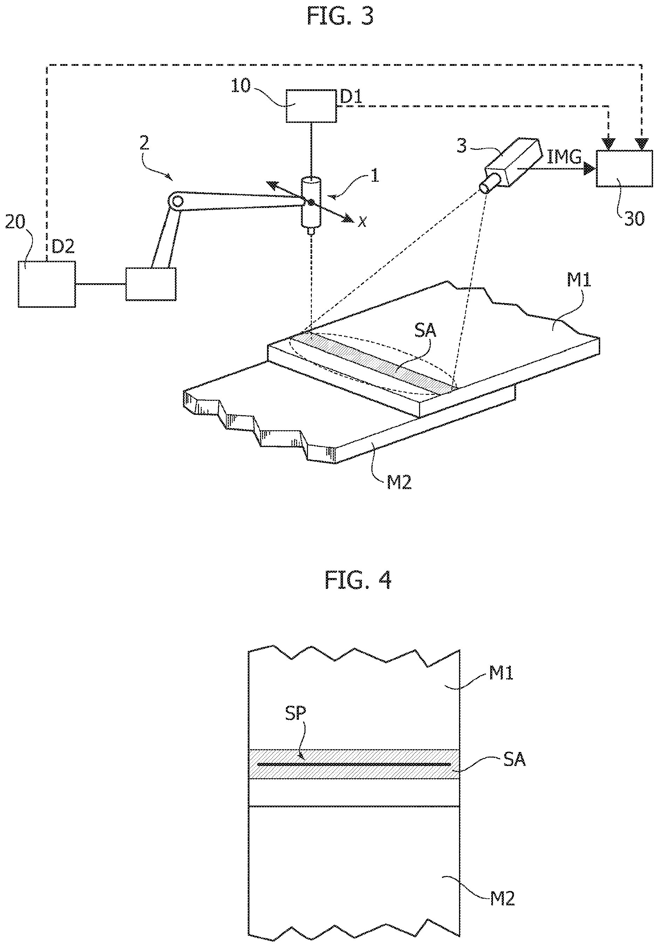

In the ensuing Figures 3 to 12, the parts, elements, or components that have already been described with reference to Figures 1 and 2 are designated by the same references used previously in the above figures. The aforesaid elements described previously will not be described again hereinafter in order not to overburden the present detailed description.

As mentioned previously, the present description provides solutions for monitoring the quality of a weld bead.

Figure 3 shows an embodiment of a welding station according to the present disclosure. The embodiment is substantially based upon the welding station described with reference to Figures 1 and 2, and the corresponding description applies entirely. In particular, also in this case, the welding station is configured for melting the material of a number of metal pieces M1 and M2 in a welding zone SA. For this purpose, the welding station comprises:

- an energy source with corresponding welding head 1, preferably a laser source, controlled via a respective control circuit 10; and
- one or more actuators 2, such as a robot arm, controlled by means of a control circuit 20 in such a way as to move the beam emitted by the welding head 1 along a welding path.

Hence, as is also shown in Figure 4, the beam emitted by the welding head 1 displaces along a welding path SP and heats the material of the overlapping pieces M1 and M2 in a welding zone SA, thereby obtaining a weld bead via melting of the materials of the pieces M1 and M2. As shown in Figure 4, in general the welding path SP does not necessarily start and end at the edges of the pieces M1 and M2. Moreover, the welding path SP may be of any shape, even though a rectilinear path developing in a direction x is preferable.

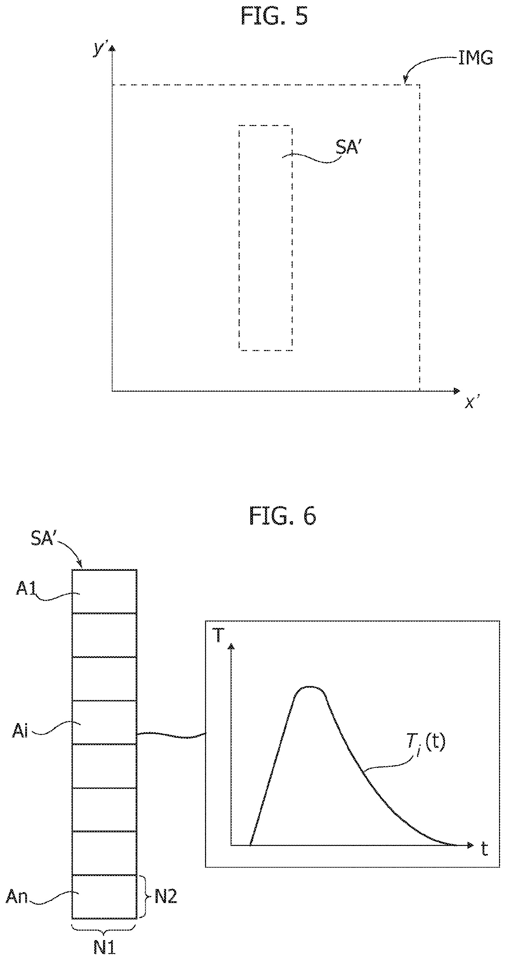

In the embodiment considered, the welding station further comprises a thermal camera 3. In particular, in various embodiments, the thermal camera 3 is mounted in a fixed position and positioned in such a way as to frame the welding zone SA; i.e., the thermal camera 3 is configured for providing a thermal image IMG that represents the welding zone SA. In general, the thermal camera 3 may be implemented also with a plurality of thermal cameras, where each thermal camera captures only a part of the welding zone SA; i.e., the image IMG may correspond to a panoramic image that results from the union of the images supplied by a plurality of thermal cameras. The thermal camera or cameras hence supplies/supply a two-dimensional image IMG in two directions x' and y' (see also Figure 5), where the value of each pixel identifies a respective temperature.

In the embodiment considered, the thermal image IMG is then processed by a processing circuit 30, such as a microprocessor programmed via software code, for example, a computer. In general, the processing circuit 30 may be implemented even together with the control circuit 10 and/or the control circuit 20 in a single electronic circuit.

For instance, Figure 5 shows schematically the thermal image IMG supplied by the thermal camera or cameras 3, where a given area SA' corresponds to the welding zone SA. Consequently, by welding together two pieces M1 and M2, the value of each pixel in the area SA' identifies the temperature of a respective point of the welding zone SA. In general, the area SA' in the image IMG may be selected manually, or else the processing circuit 30 may determine the area SA' automatically. In particular, when a welding operation is being performed, the pixels in the area SA' will have higher values, i.e., temperatures, and the processing circuit 30 can hence detect the area SA' in the image IMG that corresponds to the welding zone SA. In particular, in the case where the welding path SP is rectilinear, the area SA' will typically have a rectangular shape or, considering possible distortions of the image IMG, a trapezoidal area.

Consequently, in various embodiments, the processing circuit 30 is configured for comparing the value of each pixel of the image IMG (or of a sequence of images IMG) with a reference threshold, selecting the pixels that have a value higher than a threshold, and approximating the area in which the pixels selected are located to a rectangular or trapezoidal area.

Instead, in a currently preferred embodiment, the size of the rectangle (or trapezoidal area) is fixed. For instance, knowing the size of the welding zone SA, the processing circuit can calculate the size of the rectangle from the parameters of the thermal camera 3, for example, the focal length and the distance from the piece M1. Alternatively, the size of the rectangle (or trapezoidal area) can be set by an operator. Next, once a welding operation has been carried out, the processing circuit 30 positions the aforesaid rectangle (or trapezoidal area) in a plurality of positions in the thermal image IMG and calculates, for each position, the sum of the values of the pixels that fall within the rectangle (or trapezoidal area) for all the frames. Finally, the processing circuit 30 chooses the position/area that has the highest sum. Consequently, in this case, the processing circuit chooses as area SA' the area (of fixed size) that comprises the pixels that have as sum the maximum value, thus obtaining a compensation of minor displacements of the welding path SP for each welding operation.

As explained previously, in various embodiments, the control circuit 20 is configured for displacing, via the actuator or actuators 2, the beam emitted by the welding head 1 along a straight line in a direction x. In this case, the thermal camera or cameras 3 is/are preferably aligned in such a way that the direction x' or (as shown in Figure 5) the direction y' of the image IMG corresponds to the direction x. For instance, in this way, the rectangular area SA' is also aligned with the array of pixels of the image IMG.

Alternatively or additionally, the processing circuit 30 may process the thermal image IMG for correcting the image captured by the thermal camera 3, for example to rotate the image IMG in such a way as to align the direction x with one of the axes x' or y' of the image IMG, to compensate for the distortion of the image IMG on account of the inclination of the thermal camera 3 with respect to the surface of the piece M1 and/or the deformation of the image IMG due to the lens of the thermal camera 3. Similar operations are widely known in the context of traditional video cameras and may also be applied to images obtained from thermal cameras. For instance, as described in the document No. US 2018/0082133 A1, knowing how the camera is installed, the compensation of the distortion may be made on the basis of the information regarding the inclination of the camera.

In the embodiment considered, the processing circuit 30 then processes the values of the pixels in the area SA'.

In particular, as shown in Figure 6, in various embodiments, the processing circuit 30 divides the area SA' into a plurality of sub-areas A1, ..., An. For instance, considering that the area SA' has a width of a given number of pixels $N1$, for example in the direction x' in Figure 5, each sub-area A1, ..., An may have a width of $N1$ pixels and a height of $N2$ pixels. For instance, to increase the precision of analysis, the number of pixels N2 may be chosen between 2 and 20 pixels. Instead, to reduce the computation time, the number of sub-areas A1, ..., An may be chosen between 10 and 50, for example on the basis of the length of the weld bead/welding zone SA, and the corresponding number of pixels $N2$ may be calculated as a function of the number of sub-areas A1, ..., An chosen. Consequently, in various embodiments, the number $N2$ may be chosen, for example, between $0.2 \cdot N1$ and $2 \cdot N1$, preferably between $0.2 \cdot N1$ and $0.5 \cdot N1$.

Next, the processing circuit 30 processes the values of the pixels in each sub-area A1, ..., An to associate to each sub-area A1, ..., An a single instantaneous temperature value $Ti$. For instance, the processing circuit 30 may calculate the temperature value $Ti$ of a given sub-area Ai using, for example, the mean value or maximum value of the values of the pixels in the respective sub-area Ai. For instance, in various embodiments, the processing circuit 30 is configured for calculating the temperature value $Ti$ of a given sub-area $Ai$ via a weighted mean that associates to each pixel a weight that varies in the direction of width of the area SA' (e.g., x' in Figure 5), for instance using a lower weight for the pixels at the lateral edges of the respective sub-area Ai and a higher weight for the central pixels of the respective sub-area $Ai$.

Consequently, for each image IMG each sub-area Ai will have associated a respective temperature value $Ti$. Moreover, as shown schematically in Figure 6, by analyzing a sequence of a plurality of thermal images IMG at time $t$, i.e., a film, the processing circuit 30 can monitor the evolution of the temperature $Ti(t)$ of each sub-area $Ai$.

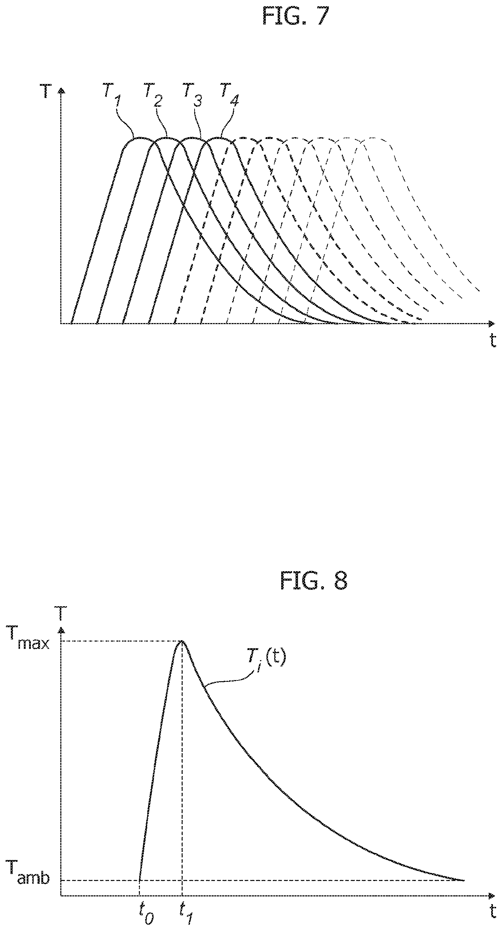

As shown in Figure 7, also considering the time necessary for following the welding path SP by means of the beam emitted by the welding head 1, the processing circuit 30 should hence analyze a plurality of temperature curves/evolutions $T1, ..., Tn$ that are staggered with respect to one another.

As illustrated in greater detail in Figure 8, each temperature evolution Ti(t) comprises:

- during a heating phase (between instants $t0$ and $t1$ in Figure 8) an increase in the temperature Ti(t) from ambient temperature $Tamb$ to a maximum temperature $Tmax$ since the respective area $Ai$ is subjected to the beam emitted by the welding head 1 to carry out welding; and
- during an immediately subsequent cooling phase (from the instant t1 in Figure 8), a reduction in the temperature $Ti(t)$ from the maximum temperature $Tmax$ towards the ambient temperature $Tamb$.

For instance, in various embodiments, the processing circuit 30 can start recording the temperatures $Ti(t)$ when the control circuit 10 supplies a trigger signal that signals start of a welding operation. Instead, the duration of recording of the temperatures $Ti(t)$ may be constant.

In general, a datum indicating the weld quality is the maximum temperature $Tmax$ reached since this datum indicates melting of the materials M1 and M2.

However, the inventors have noted that, even when the same maximum temperature *Tmax* is obtained, the profile of the cooling curve varies as a result of various welding defects, for example following upon contamination of the welding zone, for instance due to the presence of drops of water or dust.

Consequently, in various embodiments, the processing circuit 30 is configured for analyzing the cooling curve and determining a signal of status of the weld *S* as a function of the cooling curves, i.e., of the data *Ti(t)*, with *t* > *t*1, for all the sub-areas A1, ..., An.

For instance, in a first embodiment, the processing circuit 30 is configured for recording for each sub-area A1, ..., An a respective reference cooling curve during a testing step in which the weld is classified as correct (e.g., *S* = 1/OK) and then the processing circuit 30 records, during normal operation for each weld performed a respective cooling curve, and classifies the status (e.g., *S* = 1/OK or *S* = 0/NOK) of each weld by comparing the respective cooling curve recorded with the reference cooling curve. For instance, a possible solution for determining the similarity between two sequences of data and a respective classification of the similarity is described in the Italian patent application No. 102017000048962, the contents of which are incorporated herein for reference.

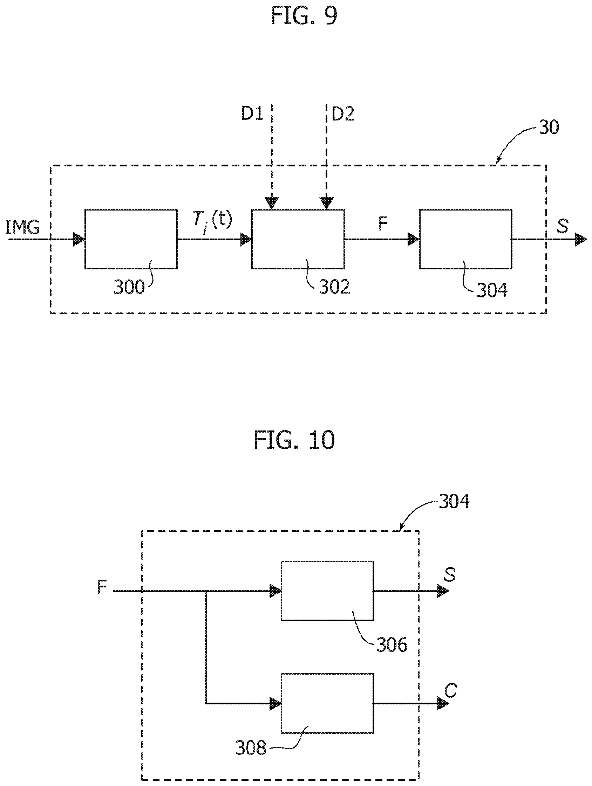

Instead, Figure 9 shows a second embodiment. In particular, in the embodiment considered, the processing circuit 30 processes, as described previously, in a pre-processing step/block 300 the sequence of images IMG supplied by the thermal camera 3 to determine a plurality of temperature curves *Ti(t)* for respective sub-areas A1, ..., An.

The above temperature curves *Ti(t)* are supplied to a step/block 302, where the processing circuit 30 processes the temperature curves *Ti(t)*. In particular, as described previously, the processing circuit 30 is configured for extracting the data of the cooling curve, for example identifying the instant t1 when the curve Ti(t) reaches a maximum value *Tmax* and selecting the data of the curve *Ti(t)* with *t* > *t*1. Next, the processing circuit 30 processes the cooling curve and determines one or more features F of the cooling curve. Consequently, step/block 302 performs a so-called feature extraction.

For instance, in various embodiments, a first feature corresponds to the maximum temperature *Tmax*. Other features F may identify the descending portion of the cooling curve, for example one or more values that indicate the time required for the temperature *Ti* to drop to a given percentage of the maximum temperature *Tmax,* for example:

- a first time $\Delta t1$ for the temperature *Ti* to drop to 750 of the temperature *Tmax;*
- a second time $\Delta t2$ for the temperature *Ti* to drop to 500 of the temperature Tmax; and
- a third time $\Delta t3$ for the temperature *Ti* to drop to 250 of the temperature *Tmax.*

Instead, in a currently preferred embodiment, the processing circuit 30 performs an operation of interpolation in order to approximate the shape of the cooling curve *Ti(t),* with *t* > *t*1, with a parameterized function. In general, this parameterized function is made up of one or more base functions, where each base function has associated a respective set of parameters. Consequently, by varying the parameters of the base functions, a combination of parameters $a_0$, ..., $a_m$ may be chosen that minimizes a cost function. For instance, the cost function may correspond to the sum of absolute differences (SAD) or the mean-squared error (MSE) calculated between the shape of the cooling curve and the parameterized function that uses the parameters chosen.

For instance, in various embodiments, a polynomial interpolation is used where the basic functions are represented by polynomials of different degree and the parameters are the coefficients of the polynomial; for example,

$$f(t) = a_0 + a_1 t + a_2 t^2 + \ldots \qquad (1)$$

Instead, in a currently preferred embodiment, an exponential interpolation is used, where the basic functions are exponential functions, for example:

$$f(t) = a_0 \cdot e^{a_1 t} + a_2 \cdot e^{a_3 t} + \ldots \qquad (2)$$

Consequently, at the end of interpolation, the processing circuit 30 chooses as features F (possibly in addition to the maximum temperature *Tmax*) the parameters $a_0$, ..., $a_m$ selected during interpolation.

In various embodiments, the circuit 30 may determine also other features F. For instance, for this purpose, step/block 302 can receive a first set of data D1 from the control circuit 10 of the source/welding head 1 and/or a second set of data D2 from the control circuit 20 of the actuator or actuators 2 (see also Figure 3). For instance, the data D1 may include the power emitted by the source and/or focusing of the welding head 1. Instead, the data D2 may include the speed of advance of the beam emitted by the welding head 1 along the welding path SP. However, also other sensors may be used and/or the processing circuit 30 may determine further features as a function of the thermal image IMG supplied by the thermal camera 3.

For instance, in various embodiments, from an analysis of the thermal image IMG, the processing circuit 30 can determine, in step 300, also dimensional parameters of the keyhole of the weld, as described for example in the document No. US 2010/0086003 A1, the contents of which is incorporated herein for reference.

For instance, in various embodiments, the above features may include, for each image IMG during the heating step, i.e., between $t0$ and $t1$, the dimensions in the directions $x'$ and/or $y'$ of the keyhole and/or a parameter that identifies distribution of the heat in the keyhole.

Additionally or alternatively, the processing circuit 30 may determine the spectral features of each image IMG, for example by means of a Fast Fourier Transform (FFT), and choose a given number of frequencies that have the maximum values.

Additionally or alternatively, the processing circuit 30 may process the last image IMG captured. In particular, the inventors have noted that, in this case, all the pixels should have substantially the same value since the pieces M1 and M2 have cooled off. However, when pieces M1 and M2 are used that are made of different materials, it is possible to note pixels that have substantially different values (i.e., higher or lower) than the average of the pixels. In particular, these pixels correspond to splashes of the material of the bottom piece M2 that have deposited on the surface of the piece M1. In particular, the above splashes may be visible, since different materials also have a different emissivity. Consequently, in various embodiments, the processing circuit 30 can determine the aforesaid pixels that seem "hotter" or "colder", for example by comparing the value of each pixel with a threshold, calculated, for instance, as a function of all the pixels of the image IMG or as a function of just a given number of pixels that surround the respective pixel. Hence, a further feature could be the number of the "hotter" or "colder" pixels.

Consequently, in general, the block/step 302 supplies a plurality of features F, where at least part of the features F identifies the shape of the cooling curves $Ti(t)$, with $t > t1$, of the sub-areas A1, ..., An.

In various embodiments, the above features F are then supplied to a step/block 304 configured for classifying the status $S$ of the weld as a function of the features F. In particular, in various embodiments, the classifier of step/block 304 is implemented with a machine-learning method.

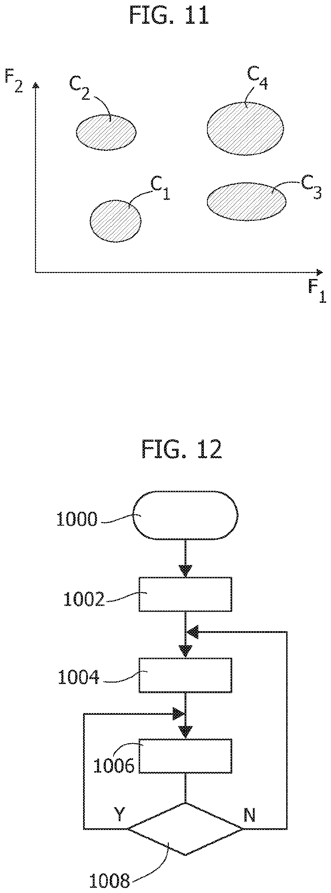

In particular, as illustrated in Figure 12, after a starting step 1000, the processing circuit 30 monitors, in a learning step 1002, a plurality of welding operations. In particular, for this purpose a plurality of welding operations are carried out under different welding conditions. For instance, for this purpose:

- the power emitted by the source and/or the focus of the welding head 1 may be varied by means of the control circuit 10; and/or
- the speed of advance of the beam emitted by the welding head 1 along the welding path SP may be varied by means of the control circuit 20; and/or
- the welding zone SA may be contaminated, for example with splashes of water and/or dust; and/or
- gripping/blocking together of the pieces M1 and M2 may be varied, for example by varying the gripping force.

Next, an operator can verify the weld quality. For instance, the operator can carry out mechanical tests (for example, tests on the strength of the connection between the pieces M1 and M2) and/or electrical tests (for example, measurements of the electrical resistance between the two pieces M1 and M2), and the operator can classify the weld quality as sufficient (for example, $S = 1$) or insufficient (for example, $S = 0$). In general, one or more of the tests used in this step may even be destructive; for example, the mechanical tests may include a tensile test in which the tensile force applied is increased up to failure of the connection between the pieces M1 and M2.

Consequently, the data acquired in step 1002 represent a training dataset, which comprises experimental data both for conditions where the weld has a sufficient quality and for conditions where the weld has an insufficient quality.

Consequently, during a training step 1004, the processing circuit 30 can extract the features F at least from the cooling curves of the sub-areas A1, ..., An (see also the description of Figure 9) and train the classifier 304 using the features F as input data of the classifier 304 and the weld status $S$ as output of the classifier 304. In general, different classifiers of the supervised-machine-learning category may be used, such as artificial neural networks or support vector machines.

For instance, in various embodiments, an artificial neural network is used, such as a network of the feed-forward type. For example, in various embodiments, such a network comprises an input layer that comprises a number of input nodes equal to the number of the features F. In addition, the network comprises a given number of hidden layers. For example, in various embodiments, the number of the hidden layers is between 2 and 5, preferably 3, and the number of nodes/neurons of each hidden layer is chosen between 1.5 and 3 times the number of the features F.

Consequently, at the end of the step 1004, the classifier 304 is able to estimate the quality of a weld as a function of a set of features F extracted at least from the shape of the cooling curves for the sub-areas A1, ..., An.

Then, once step 1004 is completed, the welding station can be used during a normal operating step 1006, where the weld quality is to be estimated without any further checks on the part of an operator.

Consequently, in step 1006, the processing circuit 30 again monitors the shape of the cooling curves (see also the description of step/block 300), determines the features F (see also the description of step/block 302), and uses the trained classifier to estimate the weld status/quality S as a function of the features F (see also the description of step/block 304).

In general, an operator can in any case carry out further tests for verifying the weld quality, as described with reference to step 1002. For instance, this may be useful during the initial step of development of a new welding process in such a way as to verify the estimate made by the classifier 304 and/or to carry out periodic monitoring of the results of the estimation, for example to obtain additional data that have not been taken into consideration previously.

Consequently, as represented schematically in Figure 12, in the case where the operator determines, in a verification step 1008, that the result of the classifier is correct (output "Y" from the verification step 1008), the process can continue with step 1006.

Instead, in the case where the operator determines, in the verification step 1008, that the result of the classifier is erroneous (output "N" from the verification step 1008), the operator can store the data of the weld made and the respective corrected quality in the training dataset and can start up the step 1004 for training the classifier again.

Consequently, in various embodiments, the data acquired during normal operation 1006 can themselves be used as training dataset. For instance, for this purpose, the processing circuit 30 may be configured, for example by means of an appropriate computer program, for storing the training dataset directly in the processing circuit 30 and managing, also directly, the training step 1004, thus enabling a new training of the classifier when the training dataset changes.

In various embodiments, the classifier 304 may be configured for supplying not only a binary result S, i.e., a result that indicates a sufficient quality or an insufficient quality, but can supply also an indication C on the type of defect. For instance, for this purpose, the operator can store (in step 1002) in the training dataset also information on a type of defect detected. For instance, such defects may correspond to the different welding conditions used in step 1002, for example an insufficient grip, impurities/contamination of the pieces M1/M2, a loss in power of the source, etc.

For instance, this is schematically illustrated in Figure 10. In particular, in the embodiment considered, the classifier 304 comprises a first classifier 306 configured for estimating the status S of the weld, which may hence be correct or defective. The output of the classifier 306 may in any case correspond to a continuous value, for example in the range between 0 and 1, which indicates the confidence of the estimate. The classifier 306 can then determine the status S as a function of the value supplied, for example assign a first value (e.g., S = 1/OK) in the case where the output value is higher than a first threshold (e.g., 0.8), or a second value (e.g., S = 1/OK) in the case where the value at output is lower than a second threshold (e.g., 0.2).

In addition or as an alternative, the classifier 304 comprises a second classifier 308 configured for estimating the defective-weld class C, which may thus present a number of values. For instance, this is schematically illustrated in Figure 11, where the values of two features F1 and F2 are mapped on four classes C1, ..., C4. In general, the number of dimensions to be considered corresponds to the number of features F taken into account.

For instance, in various embodiments, the classifier 308 comprises, for each class C, a respective output that supplies a continuous value indicating the distance of the point represented by the combination of the current values of the features F from each class C, i.e., each cluster, for example in the range between 0 and 1. For instance, in this case, the classifier 308 can choose the class C that has associated to it the highest value, possibly limiting the choice only to the clusters whereby the respective distance is less than a maximum value.

Consequently, during step 1002, the operator can determine not only the status S of the weld, but possibly also the type of defect C. In general, since the present approach is a machine-learning approach, the classifier 308 is hence able to adapt to the number of classes of defects C that the operator wishes to consider, also enabling addition of new types of defects that emerge only during normal operation 1006 of the welding station (see also the description of Figure 12). For instance, during step 1006, a situation may emerge where the weld quality becomes insufficient because the lens of the welding head 1 gets dirty, whereas this problem had not been taken into consideration during step 1002.

Of course, without prejudice to the principles underlying the invention, the details of construction and the embodiments may vary widely with respect to what has been described and illustrated herein purely to way of example, without thereby departing from the scope of the present invention, as defined by the ensuing claims.

## Claims

1. A method of analysing the quality of a weld bead in a welding zone (SA), said weld bead being generated by means of a continuous welding operation, wherein an energy beam emitted by a source with corresponding welding head (1) follows a welding path (SP), thereby melting the material of at least two or more metal pieces (M1, M2), the method comprising the steps of:

   - monitoring said welding zone (SA) via a thermal camera (3), wherein said thermal camera (3) provides a sequence of thermal images (IMG), and wherein a given area (SA') in said thermal image (IMG) corresponds to said welding zone (SA);
- dividing (300) said area (SA') into a plurality of sub-areas (A1, ..., An) and determining for each sub-area (Ai) a respective temperature (Ti) as a function of the values of the pixels within the respective sub-area (Ai);

**characterized in that** the method further comprises the steps of:

- during a learning step (1002) wherein a plurality of welding operations are performed both with sufficient quality and with insufficient quality, monitoring via said thermal camera (3) the temperature evolution ($Ti(t)$) of each sub-area ($Ai$) during each welding operation;
- during a training step (1004), processing the temperature evolutions ($Ti(t)$) monitored during said learning step for training a classifier (304) configured for estimating a weld quality as a function of respective temperature evolutions ($Ti(t)$), wherein said processing the temperature evolutions ($Ti(t)$) comprises:

- extracting (302) from each temperature evolution ($Ti(t)$) a respective cooling curve and determining for each cooling curve a plurality of parameters (F) that identify the shape of the cooling curve; and
- using said parameters (F) as input features for said classifier (304); and

- during a normal welding operating step (1006), monitoring (300) via said thermal camera (3) the temperature evolution ($Ti(t)$) of each sub-area ($Ai$) during a welding operation and estimating via said classifier (304) the respective weld quality (S, C).

2. The method according to Claim 1, wherein said determining for each cooling curve a plurality of parameters (F) that identify the shape of the cooling curve comprises approximating via interpolation the shape of the cooling curve with a function composed of a plurality of base functions, thereby selecting a set of interpolation parameters, and using said set of interpolation parameters as input features for said classifier (304).

3. The method according to Claim 2, wherein said interpolation is an exponential interpolation.

4. The method according to any one of the previous claims, wherein said determining for each sub-area (Ai) a respective temperature ($Ti$) comprises determining the temperature ($Ti$) via a mean or a weighted mean of the values of the pixels within the respective sub-area ($Ai$).

5. The method according to any one of the previous claims, comprising determining said area (SA') in said thermal image (IMG) via the steps of:

- performing a welding operation;
- defining a rectangular or trapezoidal area of interest in said thermal image (IMG); and
- positioning said area of interest in a plurality of positions in such a way as to maximize the sum of the values of the pixels in said thermal image (IMG) for a plurality of frames.

6. The method according to any one of the previous claims, wherein said classifier (304) comprises at least one artificial neural network (306, 308).

7. The method according to any one of the previous claims, using (304), in addition to said parameters (F), one or more further input features for said classifier (304), wherein said one or more further features are chosen from among:

- the maximum temperature ($Tmax$) of each temperature evolution ($Ti(t)$);
- the power emitted by said source;
- the speed of advance with which said energy beam follows said welding path (SP);
- one or more dimensional data of the keyhole produced during welding; and/or
- at the end of the welding operation, the number of the pixels in said thermal image (IMG) that has a value substantially different from the mean value of the pixels in said thermal image (IMG).

8. The method according to any one of the previous claims, comprising, following upon a normal welding operating step (1006):

- verifying the weld quality;

- comparing (1008) the weld quality estimated by said classifier (304) with the weld quality verified; and
- in the case where the weld quality estimated by said classifier (304) does not correspond to the weld quality verified, training (1004) again said classifier (304) using the temperature evolution *(Ti(t))* of each sub-area (*Ai*) monitored both during said learning step (1002) and during said normal welding operating step (1006).

9. The method according to any one of the previous claims, comprising:

- during said learning step (1002), classifying each weld that has an insufficient quality in a defective-weld class (*C*) of a plurality of classes; and
- during said training step (1004), training a classifier (308) configured for estimating a defective-weld class as a function of said temperature evolutions (*Ti(t)*).

10. A welding system, comprising:

- a source with corresponding welding head (1) configured for supplying an energy beam;
- one or more actuators (2) configured for moving said energy beam produced by said welding head (1) along a welding path (SP) in such a way as to melt the material of at least two metal pieces (M1, M2),
- a thermal camera (3); and
- a processing circuit (30) operatively connected to said thermal camera (3);

**characterized in that** said processing circuit (30) is configured for implementing the method according to any one of the previous claims.

11. A computer-program product that can be loaded into the memory of at least one processor and comprises portions of software code which, when executed by the processing circuit (30), causes the welding system according to claim 10 to execute the steps of the method according to any one of Claims 1 to 9.

**Patentansprüche**

1. Verfahren zur Analyse der Qualität einer Schweißraupe in einer Schweißzone (SA), wobei die Schweißraupe durch einen kontinuierlichen Schweißvorgang erzeugt wird, wobei ein von einer Quelle mit entsprechendem Schweißkopf (1) emittierter Energiestrahl einem Schweißpfad (SP) folgt und dabei das Material von zumindest zwei oder mehr Metallteilen (M1, M2) schmilzt, wobei das Verfahren die Schritte umfasst:

- Überwachen der Schweißzone (SA) über eine Wärmekamera (3), wobei die Wärmekamera (3) eine Folge von Wärmebildern (IMG) liefert, und wobei ein vorgegebener Bereich (SA') in dem Wärmebild (IMG) zur Schweißzone (SA) korrespondiert;
- Aufteilen (300) des Bereichs (SA') in eine Vielzahl von Unterbereichen (A1, ..., An) und Bestimmen einer jeweiligen Temperatur (Ti) für jeden Unterbereich (Ai) als eine Funktion der Werte der Pixel innerhalb des jeweiligen Unterbereichs (Ai);

**dadurch gekennzeichnet, dass** das Verfahren ferner die Schritte umfasst:

- während eines Lernschritts (1002), in dem eine Vielzahl von Schweißvorgängen sowohl mit genügender Qualität als auch mit ungenügender Qualität durchgeführt werden, Überwachen der Temperaturentwicklung (Ti (t)) jedes Unterbereichs (Ai) über die Wärmekamera (3) während jedes Schweißvorgangs;
- während eines Trainingsschritts (1004), Verarbeiten der Temperaturentwicklungen (Ti (t)), die während des Lernschritts zum Trainieren eines Klassifikators (304) überwacht werden, welcher zum Schätzen einer Schweißqualität als eine Funktion jeweiliger Temperaturentwicklungen (Ti (t)) ausgebildet ist, wobei das Verarbeiten der Temperaturentwicklungen (Ti (t)) umfasst:
- Extrahieren (302) einer jeweiligen Abkühlungskurve aus jeder Temperaturentwicklung (Ti (t)) und Bestimmen für jede Abkühlungskurve einer Vielzahl von Parametern (F), welche die Form der Abkühlungskurve identifizieren; und
- Verwenden der Parameter (F) als Eingabemerkmale für den Klassifikator (304); und
- während eines normalen Schweißvorgangsschritts (1006), Überwachen (300) der Temperaturentwicklung (Ti (t)) jedes Unterbereichs (Ai) während eines Schweißvorgangs über die Wärmekamera (3) und Schätzen der jeweiligen Schweißnahtqualität (S, C) über den Klassifikator (304).

2. Verfahren nach Anspruch 1, wobei das Bestimmen für jede Abkühlkurve einer Vielzahl von Parametern (F), welche die Form der Abkühlkurve identifizieren, ein Approximieren der Form der Abkühlkurve durch Interpolation mit einer Funktion umfasst, die aus einer Vielzahl von Basisfunktionen zusammengesetzt ist, wodurch ein Satz von Interpolationsparametern ausgewählt wird, und Verwenden des Satzes von Interpolationsparametern als Eingabemerkmale für den Klassifikator (304).

3. Verfahren nach Anspruch 2, wobei die Interpolation eine exponentielle Interpolation ist.

4. Verfahren nach einem der vorhergehenden Ansprüche, wobei das Bestimmen einer jeweiligen Temperatur (Ti) für jeden Unterbereich (Ai) ein Bestimmen der Temperatur (Ti) über einen Mittelwert oder einen gewichteten Mittelwert der Werte der Pixel innerhalb des jeweiligen Unterbereichs (Ai) umfasst.

5. Verfahren nach einem der vorhergehenden Ansprüche, umfassend ein Bestimmen des Bereichs (SA') in dem Wärmebild (IMG) über die Schritte:

   - Durchführung eines Schweißvorgangs;
   - Definieren eines rechteckigen oder trapezförmigen Bereichs von Interesse in dem Wärmebild (IMG); und
   - Positionieren des Bereichs von Interesse in einer Vielzahl von Positionen, so dass die Summe der Pixelwerte in dem Wärmebild (IMG) für eine Vielzahl von Frames maximiert wird.

6. Verfahren nach einem der vorhergehenden Ansprüche, wobei der Klassifikator (304) zumindest ein künstliches neuronales Netzwerk (306, 308) umfasst.

7. Verfahren nach einem der vorhergehenden Ansprüche, verwendend, zusätzlich zu den Parametern (F), ein oder mehrere weitere Eingabemerkmale für den Klassifikator (304), wobei das eine oder die mehreren weiteren Merkmale ausgewählt sind aus:

   - der maximalen Temperatur (Tmax) jeder Temperaturentwicklung (Ti (t));
   - der von der Quelle abgegebenen Leistung;
   - der Vorschubgeschwindigkeit, mit der der Energiestrahl dem Schweißpfad (SP) folgt;
   - einer oder mehrerer Dimensionsangaben des beim Schweißen erzeugten Schlüssellochs; und/oder
   - am Ende des Schweißvorgangs, der Anzahl der Pixel in dem Wärmebild (IMG), deren Wert sich wesentlich von dem Mittelwert der Pixel in dem Wärmebild (IMG) unterscheidet.

8. Verfahren nach einem der vorhergehenden Ansprüche, umfassend, im Anschluss an einen normalen Schweißvorgangsschritt (1006):

   - Überprüfen der Schweißqualität;
   - Vergleichen (1008) der von dem Klassifikator (304) geschätzten Schweißqualität mit der überprüften Schweißqualität; und
   - für den Fall, dass die von dem Klassifikator (304) geschätzte Schweißqualität nicht mit der überprüften Schweißqualität übereinstimmt, erneutes Training (1004) des Klassifikators (304) unter Verwendung der Temperaturentwicklung (Ti (t)) jedes Unterbereichs (Ai), die sowohl während des Lernschritts (1002) als auch während des normalen Schweißvorgangsschritts (1006) überwacht wird.

9. Verfahren nach einem der vorhergehenden Ansprüche, umfassend:

   - während des Lernschritts (1002), Klassifizieren jeder Schweißnaht, die eine ungenügende Qualität aufweist, in eine Klasse (C) fehlerhafter Schweißnähte von einer Vielzahl von Klassen; und
   - während des Trainingsschritts (1004), Trainieren eines Klassifikators (308), der zum Schätzen einer Klasse fehlerhafter Schweißnähte als eine Funktion der Temperaturentwicklungen (Ti (t)) ausgebildet ist.

10. Schweißsystem, umfassend:

   - eine Quelle mit entsprechendem Schweißkopf (1), die zur Bereitstellung eines Energiestrahls ausgebildet ist;
   - ein oder mehrere Aktuatoren (2), die ausgebildet sind, den von dem Schweißkopf (1) erzeugten Energiestrahl entlang eines Schweißpfads (SP) zu bewegen, so dass das Material von zumindest zwei Metallteilen (M1, M2) schmilzt,

- eine Wärmekamera (3); und
- eine Verarbeitungsschaltung (30), die operativ mit der Wärmekamera (3) verbunden ist;

**dadurch gekennzeichnet, dass** die Verarbeitungsschaltung (30) zur Implementierung des Verfahrens nach einem der vorherigen Ansprüche ausgebildet ist.

11. Computerprogrammprodukt, das in den Speicher von mindestens einem Prozessor ladbar ist und Teile von Softwarecode umfasst, der, wenn von der Verarbeitungsschaltung (30) ausgeführt, das Schweißsystem nach Anspruch 10 veranlasst, die Schritte des Verfahrens nach einem der Ansprüche 1 bis 9 auszuführen.

**Revendications**

1. Procédé d'analyse de la qualité d'un cordon de soudure dans une zone de soudage (SA), ledit cordon de soudure étant généré au moyen d'une opération de soudage en continu, dans lequel un faisceau d'énergie émis par une source à tête de soudage correspondante (1) suit un chemin de soudure (SP), faisant ainsi fondre le matériau d'au moins deux pièces métalliques ou plus (M1, M2), le procédé comprenant les étapes de :

- surveillance de ladite zone de soudage (SA) par l'intermédiaire d'une caméra thermique (3), dans laquelle ladite caméra thermique (3) fournit une séquence d'images thermiques (IMG), et dans laquelle une région donnée (SA') dans ladite image thermique (IMG) correspond à ladite zone de soudage (SA) ;
- division (300) de ladite région (SA') en une pluralité de sous-régions (A1, ..., An) et détermination pour chaque sous-région (Ai) d'une température respective (Ti) en fonction des valeurs des pixels dans la sous-région respective (Ai) ;

**caractérisé en ce que** le procédé comprend en outre les étapes de :

- lors d'une étape d'apprentissage (1002) dans laquelle une pluralité d'opérations de soudage sont réalisées à la fois avec une qualité suffisante et avec une qualité insuffisante, surveillance par l'intermédiaire de ladite caméra thermique (3) de l'évolution de température (Ti(t)) de chaque sous-région (Ai) lors de chaque opération de soudage ;
- lors d'une étape d'entraînement (1004), traitement des évolutions de température (Ti(t)) surveillées lors de ladite étape d'apprentissage pour entraîner un classificateur (304) configuré pour estimer une qualité de soudure en fonction d'évolutions de température respectives (Ti(t)), dans laquelle ledit traitement des évolutions de température (Ti(t)) comprend :

  - l'extraction (302) de chaque évolution de température (Ti(t)) d'une courbe de refroidissement respective et la détermination pour chaque courbe de refroidissement d'une pluralité de paramètres (F) qui identifient la forme de la courbe de refroidissement ; et
  - l'utilisation desdits paramètres (F) comme caractéristiques d'entrée pour ledit classificateur (304) ; et

- lors d'une étape de fonctionnement normal de soudage (1006), surveillance (300) par l'intermédiaire de ladite caméra thermique (3) de l'évolution de température (Ti(t)) de chaque sous-région (Ai) lors d'une opération de soudage et estimation par l'intermédiaire dudit classificateur (304) de la qualité de soudure respective (S, C).

2. Procédé selon la revendication 1, dans lequel ladite détermination pour chaque courbe de refroidissement d'une pluralité de paramètres (F) qui identifient la forme de la courbe de refroidissement comprend l'approximation par interpolation de la forme de la courbe de refroidissement avec une fonction composée d'une pluralité de fonctions de base, sélectionnant ainsi un ensemble de paramètres d'interpolation, et utilisant ledit ensemble de paramètres d'interpolation comme caractéristiques d'entrée pour ledit classificateur (304).

3. Procédé selon la revendication 2, dans lequel ladite interpolation est une interpolation exponentielle.

4. Procédé selon l'une quelconque des revendications précédentes, dans lequel ladite détermination pour chaque sous-région (Ai) d'une température respective (Ti) comprend la détermination de la température (Ti) par l'intermédiaire d'une moyenne ou d'une moyenne pondérée des valeurs des pixels dans la sous-région respective (Ai).

5. Procédé selon l'une quelconque des revendications précédentes, comprenant la détermination de ladite région (SA') dans ladite image thermique (IMG) par les étapes de :

- réalisation d'une opération de soudage ;
- définition d'une région d'intérêt rectangulaire ou trapézoïdale dans ladite image thermique (IMG) ; et
- positionnement de ladite région d'intérêt dans une pluralité de positions de manière à maximiser la somme des valeurs des pixels dans ladite image thermique (IMG) pour une pluralité de trames.

6. Procédé selon l'une quelconque des revendications précédentes, dans lequel ledit classificateur (304) comprend au moins un réseau neuronal artificiel (306, 308).

7. Procédé selon l'une quelconque des revendications précédentes, utilisant (304), en plus desdits paramètres (F), une ou plusieurs caractéristiques d'entrée supplémentaires pour ledit classificateur (304), dans lequel lesdites une ou plusieurs caractéristiques supplémentaires sont choisies parmi :

- la température maximale ($Tmax$) de chaque évolution de température ($Ti(t)$) ;
- la puissance émise par ladite source ;
- la vitesse d'avancement avec laquelle ledit faisceau d'énergie suit ledit chemin de soudure (SP) ;
- une ou plusieurs données dimensionnelles du trou de serrure produit lors du soudage ; et/ou
- à la fin de l'opération de soudage, le nombre de pixels dans ladite image thermique (IMG) qui présente une valeur sensiblement différente de la valeur moyenne des pixels dans ladite image thermique (IMG).

8. Procédé selon l'une quelconque des revendications précédentes, comprenant, suite à une étape de fonctionnement normal de soudage (1006) :

- la vérification de la qualité de soudure ;
- la comparaison (1008) de la qualité de soudure estimée par ledit classificateur (304) avec la qualité de soudure vérifiée ; et
- dans le cas où la qualité de soudure estimée par ledit classificateur (304) ne correspond pas à la qualité de soudure vérifiée, l'entraînement (1004) à nouveau dudit classificateur (304) en utilisant l'évolution de température $(Ti(t))$ de chaque sous-région ($Ai$) surveillée à la fois lors de ladite étape d'apprentissage (1002) et lors de ladite étape de fonctionnement normal de soudage (1006).

9. Procédé selon l'une quelconque des revendications précédentes, comprenant :

- lors de ladite étape d'apprentissage (1002), la classification de chaque soudure qui présente une qualité insuffisante dans une classe de soudure défectueuse (C) d'une pluralité de classes ; et
- lors de ladite étape d'entraînement (1004), l'entraînement d'un classificateur (308) configuré pour estimer une classe de soudure défectueuse en fonction desdites évolutions de température $(Ti(t))$.

10. Système de soudage, comprenant :

- une source avec une tête de soudage correspondante (1) configurée pour fournir un faisceau d'énergie ;
- un ou plusieurs actionneurs (2) configurés pour déplacer ledit faisceau d'énergie produit par ladite tête de soudage (1) le long d'un chemin de soudure (SP) de manière à faire fondre le matériau d'au moins deux pièces métalliques (M1, M2),
- une caméra thermique (3) ; et
- un circuit de traitement (30) connecté fonctionnellement à ladite caméra thermique (3) ;

**caractérisé en ce que** ledit circuit de traitement (30) est configuré pour mettre en oeuvre le procédé selon l'une quelconque des revendications précédentes.

11. Produit de programme informatique qui peut être chargé dans la mémoire d'au moins un processeur et comprend des parties de code logiciel qui, lorsqu'il est exécuté par le circuit de traitement (30), amène le système de soudage selon la revendication 10 à exécuter les étapes du procédé selon l'une quelconque des revendications 1 à 9.

FIG. 1

FIG. 2A

FIG. 2B

FIG. 2C

# FIG. 3

# FIG. 4

## FIG. 5

## FIG. 6

## FIG. 7

## FIG. 8

## FIG. 9

## FIG. 10

# FIG. 11

# FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150207127 A1
- US 20170341144 A1
- DE 102013112244 A1
- US 20180082133 A1
- IT 102017000048962
- US 20100086003 A1

### Non-patent literature cited in the description

- **DAS, A. ; LI, D ; WILLIAMS, D ; GREENWOOD, D.** Joining Technologies for Automotive Battery Systems Manufacturing. *World Electr. Veh. J.,* 2018, vol. 9, 22